# EUROPEAN PATENT SPECIFICATION

(11) **EP 2 724 670 B1**
(45) Date of publication and mention of the grant of the patent: **17.06.2015**
(21) Application number: 12190074.0
(22) Date of filing: 26.10.2012
(51) Int. Cl.: A61B 5/145, A23K 1/00, A23K 1/18, A61K 9/00

(54) **Detecting altered pH levels of rumens**
Erkennung veränderter pH-Werte von Pansen
Détection des niveaux de pH modifiés de rumens

(43) Date of publication of application: 30.04.2014
(73) Proprietor: Kankfelt, Pekka, 46230 Valkeala (FI)
(72) Inventor: Kankfelt, Pekka, 46230 Valkeala (FI)
(74) Representative: Kolster Oy Ab

(56) References cited:
- US-A- 4 473 545
- US-A1- 2002 128 542
- US-A1- 2008 160 084
- US-A1- 2011 268 792
- MYERS Z H ET AL: "Evaluating estimates of phosphorus maintenance requirement of lactating Holstein cows with different dry matter intakes", JOURNAL OF DAIRY SCIENCE, AMERICAN DAIRY SCIENCE ASSOCIATION, US, vol. 92, no. 2, 1 February 2009 (2009-02-01), pages 708-719, XP026955252, ISSN: 0022-0302 [retrieved on 2009-02-01]

## Description

### FIELD OF THE INVENTION

The present invention relates to a method for detecting acidosis in a rumen of a ruminant and discloses optimizing feeding of a ruminant. The present invention also relates to a device for detecting acidosis in the rumen of a ruminant, to a use of the device for detecting acidosis in the rumen of a ruminant and to animal feed.

### BACKGROUND OF THE INVENTION

The word "ruminant" comes from the Latin *ruminare*, which means "to chew over again". The ruminants have a four-compartment stomach (rumen, reticulum, omasum, and abomasum). The rumen and reticulum are called the reticulorumen and represent the same functional space as digesta can move back and forth between them. The rumen, also known as a paunch, forms the larger part of the reticulorumen, which is the first chamber in the alimentary canal of ruminant animals. It serves as the primary site for microbial fermentation of ingested feed. The smaller part of the reticulorumen is the reticulum, which is fully continuous with the rumen, but differs from it with regard to the texture of its lining.

The degraded digesta then passes into the omasum, where water and many of the inorganic mineral elements are absorbed into the blood stream. After this the digesta is moved to the true stomach, the abomasum. The abomasum, also known as the maw, rennet-bag or reed tripe, is the fourth and final stomach compartment in ruminants. Digesta is finally moved into the small intestine, where the digestion and absorption of nutrients occurs. Fermentation continues in the large intestine in the same way as in the reticulorumen.

Microbes in the reticulorumen eventually flow out into the omasum and the remainder of the alimentary canal. Under normal fermentation conditions the environment in the reticulorumen is weakly acidic and is populated by microbes that are adapted to a pH between roughly 5.5 and 6.5; since the abomasum is strongly acidic (pH 2 to 4), it acts as a barrier that largely kills reticulorumen flora and fauna as they flow into it. Subsequently, microbial biomass is digested in the small intestine and smaller molecules (mainly amino acids) are absorbed and transported in the portal vein to the liver. The digestion of these microbes in the small intestine is a major source of nutrition, as microbes usually supply some 60 to 90% of the total amount of amino acids absorbed. On starch-poor diets, they also provide the predominant source of glucose absorbed from the small intestinal contents. Under conditions of ruminal acidosis, when the environment of the reticulorumen has become too acidic (usually due to excessive fermentation of starches and sugars into VFA and lactate), microbes that favor a lower pH may start to dominate the ecosystem of the reticulorumen. This gives rise to rumen acidosis and often feed intake of the ruminant will drop.

One of the most common causes of acidosis occurs when switching from a high fiber to high concentrate diet that is rich in fermentable carbohydrates (starches and sugars). Large amounts of starch and sugar stimulate bacteria that make lactic acid. In this instance, bacteria that normally use lactic acid cannot keep up with production. The level of acidity in the rumen is measured by pH readings. The optimal rumen pH is between 6.0 and 6.2, but there is daily fluctuation below this level even in healthy cows. Lactic acid is about ten times a stronger acid than the other rumen acids and causes the rumen pH to decrease. As the rumen pH drops below 6.0, bacteria that digest fiber begin to die and thus, fiber digestion is depressed. Because the end products of fiber digestion are used for milk fat synthesis, a drop in milk fat test is a sure sign of acidosis. In, addition, the accumulation of acid causes an influx of water from the tissues into the gut and thus a common sign of acidosis is diarrhea. If the rumen pH continues to decline and falls below 5.5, many other normal healthy rumen bacteria also begin to die. As lactic acid accumulates, it is absorbed and lowers the pH of the blood. High levels of acid in the gut can also cause ulcers in the rumen resulting in infiltration of bacteria into the blood that can cause liver abscesses. Endo-toxins resulting from high acid production in the rumen also affects blood capillaries in the hoof, causing them to constrict resulting in laminitis. Sub-acute acidosis is also characterized by cycling intake because animals eat less during times of distress, then if the rumen adapts, their appetite returns. If blood pH drops too low, this can result in death of the animal in acute acidosis.

Another common cause of acidosis is having diets too low in effective fiber or too small particle size. When animals don't chew their cud normally, lack of saliva (that contains a natural buffer) contributes to low rumen pH. Also, some mycotoxins can alter the metabolism of lactic acid causing it to build up and cause acidosis. This may explain why acidosis and laminitis are also commonly observed when mycotoxins are a problem.

Two types of rumen acidosis are reported in the field: acute and subacute acidosis. Acute acidosis is less common and severe. Affected animals are depressed, off-feed, elevated heart rate, diarrhea, and may die. Cows experiencing subacute rumen acidosis have mild diarrhea, lower dry matter, and hemorrhages in the hoof. Rumen pH drops below 6 and remain low for several hours and VFA (volatile fatty acids) patterns shift (higher levels of propionate with an acetate to propionate ratio < 2.2).

As the functioning of the rumen is very important for the quality and quantity of milk production, all malfunctions of the rumen are to be avoided in milk related agriculture.

In order to know the state of rumen, typically, pH of the rumen liquid is measured. Acidosis of the ruminant can be detected by oral sampling of rumen liquid or by puncture to the stomach using an injection needle. However, these detection methods have either a problem of saliva mixed to the rumen liquid or a possibility of bacterial infection caused by an injection needle. Furthermore, there are different devices utilizing electric power for detecting internal state of a rumen of a ruminant (e.g. US2012088988, US2002128542, WO0113712, GB2455700, WO11079338). Usually these devices include a magnet for taking out the detection device from the body. Usually these devices or instruments also include a monitoring system outside of the body of an animal for collecting the data and for monitoring the state of the rumen by the data.

However, the devices used for detecting the pH of the rumen are expensive, their use is complicated and time consuming and usually, the devices must also be placed into the stomach of a ruminant. Overall, systems related to pH measurements comprise several components.

Studies on the digestive tract of ruminants are well known to a person skilled in the art. For example Myers Z.H. and Beede D.K. (2009, J. Dairy Sci. 92:708-719) have studied the role of phosphorus in lactating dairy cows e.g. by measuring fecal phosphorus excretion.

Also, in general, use of coloring agents or compositions having modulated release properties, are well known to a person skilled in the art. For example US20080160084 A1 describes coloring agents and natural water-insoluble encapsulation compositions suitable e.g. for animal consumption and US20110268792 A1 describes a method for the preparation of a solid application form of a medicament with modulated release characteristics *in vivo,* utilizing surface active proteins.

### BRIEF DESCRIPTION OF THE INVENTION

An object of the present invention is to provide a method and a device to solve the above problems or to alleviate the above disadvantages. The objects of the invention are achieved by methods and a device, which are characterized by what is stated in the independent claims.

The invention is based on the idea of simply detecting acidosis of a rumen by detecting visible changes of the excrement. The invention is based on the realization that by colouring agents, which are released in the digestive tract of the ruminant due to continued low pH values, changes of the pH values can be observed.

The present disclosure relates to a method for detecting acidosis in a rumen of a ruminant, the method comprising the following steps:
i) providing a device for a ruminant, the device comprising at least one colouring agent which is released in the rumen when the pH value of the rumen is less than 6 for a prolonged time, and
ii) detecting a visible change of the excrement when the ruminant has acidosis in a rumen.

Specifically, the present invention relates to a method for detecting acidosis in the rumen of a ruminant, the method comprising the following steps:
i) providing a device for a ruminant, the device comprising at least one colouring agent and a protective agent adapted to be dissolved in the rumen when the pH value of the rumen is less than 6 for at least 3 hours, so that the coloring agent is released, and
ii) detecting a visible change of the excrement when the ruminant has acidosis in the rumen.

Furthermore, the present disclosure relates to a method for optimizing feeding of a ruminant, the method comprising the following steps:
i) providing a device for a ruminant, the device comprising at least one colouring agent which is released in the rumen when the pH value of the rumen is less than 6 for a prolonged time,
ii) detecting a visible change of the excrement when the ruminant has an acid rumen, and
iii) changing the diet of a ruminant having a visible change of the excrement.

Furthermore, the present disclosure relates to a device for detecting acidosis in a rumen of a ruminant, comprising
a) a colouring agent, which is released in a rumen in acidic conditions when a pH value is less than 6 for a prolonged time, causing a visible change of excrement, and
b) a protective agent which dissolves in a rumen in acidic conditions when a pH value is less than 6 for a prolonged time.

Specifically, the present invention relates to a device for detecting acidosis in the rumen of a ruminant, wherein the device comprises
a) a colouring agent adapted to cause a visible change of the excrement, and
b) a protective agent which is adapted to be dissolved in the rumen in acidic conditions when the pH value is less than 6 for at least 3 hours, thereby releasing the colouring agent.

Still, the present invention relates to use of a device of the invention for detecting acidosis in the rumen of a ruminant.

And still, the present invention relates to animal feed comprising the capsule of the invention.

Indicating acidosis, preferably subacute acidosis, or any changes in the pH ranges of rumens of the ruminants is a challenge. Therefore, the present invention provides a low-price, simple and natural application for detecting pH changes of the rumen of the ruminant. Furthermore, the application of the present invention can be used continuously, and the ecological aspects are also taken into account.

Controlling rumen acidosis by utilizing rumen pH measurements is important for maintaining health of the ruminants and for satisfactory production of milk. For feeding management of both milk cows and beef cattle, it is important to know the state of rumens. It is easy to change the diet of the animals based on the indication method of the present invention, if pH changes occur. It is therefore possible to influence the content of the diet quickly after pH changes.

### DETAILED DESCRIPTION OF THE INVENTION

### ACIDOSIS

Fiber digesting bacteria growth is favored with a pH from 6.0 to 6.8 while starch digesting bacteria growth is favored by a pH from 5.5 to 6. Thus, the high producing cows must maintain a pH near 6.0 for optimal growth of both bacteria populations resulting in a favorable VFA pattern and yield. Several factors impact changes in rumen pH.

The type of diet can shift pH with high forage rations favoring a pH over 6. Forages stimulate higher rates of saliva secretion which contains bicarbonate which buffers the rumen and increases acetate production. The carbohydrate in forage (cellulose and hemicellulose) is not degraded as rapidly by the rumen microbes as are carbohydrates in concentrates (starch and sugar). Legume forages also have a higher natural buffering capacity.

Physical form of feeds (grinding, pelleting, and chopping) will change the size of the feed particle. If forage particle size is too short, a forage mat in the rumen is not be maintained, fiber digestion decreased, and lower rumen pH lowered. Saliva production is also reduced due to less cud chewing time. Cows will typically spend over 500 minutes of chewing time per day, 12 to 15 minutes of chew time per pound of dry matter, and 50 percent of the cows chewing their cuds when resting. If concentrates are ground too fine, starch is exposed to microbial digestion and increased degradation. Rumen pH drops and propionic acid production increases changing milk components (less milk fat percentage and higher milk protein percentage) and lower milk yield. Steam flaking, pelleting, or grinding will change starch structure (more available in the rumen for fermentation) which can be beneficial (increases rumen microbial growth) or negative (increases the risk of rumen acidosis).

Level of feed intake changes rumen degradation and synthesis. Rumen pH can drop as more substrate (such as starch) is available for microbial use increasing acid production (negative effect). The amount of saliva produced per unit of dry matter can also decline.

Wet rations can reduce rumen pH due to less saliva production to wet the feed for swallowing. If the wet feed is silage, less chewing is needed to reduce particle size lowering rumination time. Silage can have a pH below 4 increasing acid load. Adding sodium bicarbonate to corn silage raising pH above 5 increased intake prior to feeding. If the total ration dry matter exceeds 50 percent due to ensiled and fermented feeds, dry matter intake can be reduced.

Adding unsaturated fats and oils (such as vegetable and fish oils) can reduce rumen pH and shift VFA patterns. Unsaturated fatty acids can reduce fiber digestibility, decrease rumen pH, be toxic to fiber digesting bacteria, and/or coat fiber particles reducing fiber digestion. Processing of oilseeds (such as grinding or extruding) can rupture the cell wall of the seed and releasing the oil in the rumen. Feeding whole oil seeds can reduce this risk.

The method of feeding will change the rumen environment. TMR (total mixed rations) stabilizes rumen pH, synchronizes DIP and fermentable carbohydrate, increases dry matter intake, and minimizes feed selection. If concentrates are fed separately, their amount should be limited, high levels of starch-containing grains should be avoided, and the effect of feed processing should be evaluated.

In the present invention, the methods and devices utilize the condition of a rumen, when a pH value is less than 6 for a prolonged time. In a preferred embodiment of the invention, a prolonged time is from about 2 to about 5 hours. Prolonged time can also be selected from a group consisting of from about 3 hours to about 10 hours, from about 3 hours to about 8 hours and from about 4 hours to about 6 hours. In another embodiment of the invention, a prolonged time is selected from a group consisting of at least 3 hours, from 3 to 5 hours, from 3 to 6 hours, from 3 to 7 hours, from 3 to 8 hours, from 3 to 9 hours, from 3 to 10 hours, from 3 to 11 hours, from 3 to 12 hours, from 3 to 20 hours or from 3 to 24 hours.

When pH of a rumen changes to a value of less than 6, the diet of the ruminants should be changed in order to prevent symptoms of the acidosis and to guarantee continued milk production. In a preferred embodiment of the invention, changing the diet comprises at least one selected from a group consisting of changing to fiber rich diet, changing diet to bigger particle size, changing to diet containing less carbohydrates, decreasing unsaturated fats and oils, and changing the method of feeding. Alternative to changing diet, ruminants with visible change of the excrement due to acidosis can be given medicine or some buffer to neutralize the pH of the rumen.

### PROTECTIVE AGENT

EP2460415 A1 reports a preparation suitable for release of the cashew nut shell liquid in a rumen of the ruminant. The coated formulation of the invention is used as a rumen fermentation improving agent for ruminants, a bloat controlling agent, a bloat therapeutic agent, an acidosis therapeutic agent, an agent for controlling a disease caused by Clostridium, an agent for controlling a disease caused by Coccidia, or a body weight gain agent for a domestic animal. EP2460415 A1 does not report any detection of an acidosis.

Different medicinal preparations or nutrients utilizing special coatings for releasing medicine or other active agents based on low pH conditions of the digestive tract are known in the prior art (US3829564, US3880990, US4713245**,** US4473545**,** JP59198946). However, none of these documents describes detection of acidosis of the rumen before the animal has visible symptoms.

Protective agents used in the present invention can be coating materials capable of forming a continuous film around the core. On the other hand, the capsule or bolus of the invention may also be made only out of protective agent and coloring agent. The protective agent has the ability to withstand environmental conditions of the rumen, and the ability to expose the core material, if any, of the pellet in the environment of the rumen. Thus, the protective agent should be resistant to pH conditions of greater than 6 The protective agent should release the core material, if present, after exposure to rumen environmental conditions having a pH of less than 6 for a prolonged period. Release should occur within the residence time in the rumen after contacting pH about less than 6.

Another requirement for the protective agent is its ability to withstand mechanical abrasion because in case the device of the present invention is given to the ruminant together with feed, the device must enter the digestive tract without being damaged by the teeth of the ruminant.

The protective agent according to this invention comprises a mixture or blend of at least one polymeric substance. In addition, at least one further component may be selected from at least one hydrophobic substance and at least one flake material (such as plastic flakes). The hydrophobic substance and flake material are normally dispersed in the polymeric matrix.

Examples of the protective agent used in the present invention include zein (manufactured by Kobayashi Perfumery Co., Ltd.), shellac (manufactured by Gifu Shellac manufacturing Co., Ltd.), HPMC (hydroxypropyl methylcellulose: product name Metolose, manufactured by Shin-Etsu Chemical Co., Ltd.), pullulan (manufactured by Hayashibara Shoji, Inc.), and hemilose (manufactured by Freund), a sugar such as sucrose, lactose, and torehalose, a water-soluble sheet such as an oblate and a water-soluble paper but the agent is not limited thereto.

In a preferred embodiment of the invention, a protective agent forms a coating surrounding a colouring agent(s) or a protective agent is dyed throughout with a colouring agent. Protective agent(s) may essentially form the device, e.g. capsule or bolus, of the present invention. Any known methods of the prior art can be used for forming a coating or dying a protective agent.

### Polymeric substances

In a preferred embodiment of the invention, a protective agent comprises a polymer, copolymer or mixture thereof. In another embodiment of the invention, a protective agent comprises polymers, copolymers or mixtures thereof. Mixtures of different polymers and/or copolymers may also be utilized.

Examples of suitable polymeric substances include modified natural polymers, homo- and interpolymers, homo- and copolymers and mixtures thereof. The polymeric material is comprised of at least one polymer, copolymer, or blend of polymers selected from the group consisting of cellulose derivatives such as cellulose propionate morpholinobutyrate; containing addition-type monomeric moieties such as acrylonitrile; vinylated derivatives of pyridine; styrene; methylstyrene; vinyl toluene; esters and amides of methacrylic acid; acrylic acid; such as a dialkylamino ethyl acrylate or methacrylate in which the alkyl group contains from 1 to 6 carbon atoms, polymerizable ethylenically unsaturated aliphatic hydrocarbon monomers such as ethylene, propylene or butadiene; vinyl esters such as vinyl acetate, vinyl propionate or vinyl stearate; vinyl esters such as methyl, ethyl, propyl or stearyl, vinyl substituted heterocyclic ring or condensed ring compounds containing basic nitrogen configurations such as vinyl carbazole, vinyl quinoline, N-vinylpyrrole and 5-vinyl pyrozoline.

Especially preferred are poly(vinylpyridine), polymeric derivatives of vinyl-pyridine, and the copolymers of the various isomers and derivatives of vinylpyridine copolymerized with one or more of the above-mentioned addition type monomers.

These polymers, copolymers or mixtures thereof may be commercially available or may be produced by conventional techniques well known in the art.

It is important that the polymer and hydrophobic substance, if present, have a degree of compatibility to permit the protective agent to remain intact in the rumen environment when the pH value is about 6 or more for long times.

### Hydrophobic substances

Hydrophobic substances as a dispersed phase in the protective polymer are used to reduce wetting of the protective polymer and therefore attack by water, to reduce total volume of the protective polymer affected by water, and to extend the length of permeable pathway the water must travel.

Suitable hydrophobic substances include e.g. fatty acids having from 12 to 32 carbon atoms, such as oleic acid and stearic acid, dimer acids, trimer acids, aluminum salts of fatty acids, waxes, resins, and certain polymers such as polymers containing very hydrophobic chemical groups such as silicone moieties and certain multivalent cation soaps. The hydrophobic substance may be amorphous or crystalline and preferably essentially dispersible in the coating solvent when a solvent is used in which case it should not contribute significantly to the solution viscosity. Aluminum salts of such acids, for example, aluminum oleates, aluminum stearates, aluminum dimerates, are also useful. Also, the hydrophobic material may be one or more polycarboxylic acids having a ratio of from 10 to 22 carbon atoms per carboxyl group and a molecular weight greater than 300, preferably about 400 to about 1000, are useful. Blends of these acids and/or sales are also useful.

### Other substances

Physiologically acceptable flake materials may also be dispersed throughout the polymeric matrix. The flake material is substantially inert with respect to the environment of the rumen. Suitable inert flake materials include metal flake, mineral flake, crosslinked organic polymer, etc. Especially suitable are aluminum flake, talc, graphite, and ground mica.

All materials of the invention i.e. at least a protective agent(s) and a coloring agent(s) are physiologically acceptable, i.e., the materials do not interfere with the ruminants' normal body functioning.

### COLORING AGENT

A coloring agent may be any agent that retains its coloring properties when carried along the digestive tract. Any conditions of the tract, for example enzymes and different pH conditions, may not remove or eliminate the color. In other words, if the color has been released by a capsule or bolus, a visible effect must be seen in the excrement of the ruminants.

A coloring agent used in the present invention may be in any form such as a liquid, powder, gel or paste. In a preferred embodiment of the invention, the coloring agent has a strong or exceptional color such as red or violet.

The coloring agent may for example be a food coloring, or color additive, i.e. any dye, pigment or substance that imparts color when it is added to a food or drink. The advantages of food colors are safety and general availability. The coloring agent of the present invention must be safe for animals.

The coloring agents may also be pH dependent, such as anthocyanins (water-soluble vacuolar pigments that may appear red, purple, or blue depending on the pH). If the coloring agent changes its color when carried along the digestive tract, it still must provide visible effect in the excrement of the ruminants. Furthermore, radiation such as UV-light may cause visible effects to coloring agents in the excrement of the ruminant after defecation has occurred.

It is possible to have only one or several different coloring agents in a device of the present invention.

Coloring agents may also be added to any material comprised in the device of the present invention. For example colored inert plastic flakes may provide the visible effect of the excrement.

### DEVICES

In methods of the present invention, the device is given to the animal. This is done either by giving the device (e.g. capsule) together with the feed to the ruminant or the device (e.g. bolus) is installed into the rumen of the animal.

In a preferred embodiment of the invention a device is a capsule for oral administration to a ruminant, the capsule comprising a colouring agent and a protective agent which dissolves in a rumen when a pH value is less than 6 for a prolonged time.

In a more preferred embodiment, a device is a capsule for oral administration to a ruminant, the capsule comprising a colouring agent and a protective agent which dissolves in a rumen within about 2 to about 5 hours when a pH value is less than 6.

In another preferred embodiment of an invention a device is a bolus for a rumen of a ruminant, the bolus comprising a colouring agent, which is released to a rumen when a pH value is less than 6.

In a further preferred embodiment of an invention a device is a bolus for a rumen of a ruminant, the bolus comprising a colouring agent and a protective agent which dissolves in a rumen when a pH value is less than 6 for a prolonged time. The bolus is installed to a rumen of the ruminant, and it is expected to serve for the purpose of indicating acidosis for several days, weeks or months.

The feed may stay in the rumen for about 12-24 hours and during that time the device releases a coloring agent(s) to the rumen if a pH value is less than 6 for a prolonged time.

It is also possible to include medicaments or other chemical agents in a device of the present invention for alleviating symptoms of acidosis. For example, sodium bicarbonate is preferred for buffering the acid conditions of the rumen.

### ANIMAL FEED

Animal feed of the present invention is not particularly limited as long as it comprises the protective agent, but the animal feed may further contain any arbitrary component such as a component which is effective for the growth promotion of a ruminant, a nutritional supplement component, or a component for enhancing the preservation stability.

The methods and devices of the present invention are suitable for ruminants such as cows, goats, and sheep.

In a preferred embodiment of the invention, animal feed is a pellet adapted for oral administration to a ruminant.

Capsules of the present invention are preferably given in every day basis to the ruminants, allowing continuous detection of the pH values in the rumen. On the other hand, it is possible to use capsules only when the pH values are wanted to be checked.

It will be obvious to a person skilled in the art that, as the technology advances, the inventive concept can be implemented in various ways. The invention and its embodiments are not limited to the examples described above but may vary within the scope of the claims.

The present invention is illustrated by the following examples, which are not intended to be limiting in any way.

### EXAMPLE 1.

The cow is given pellets together with feed, each pellet comprising a capsule comprising a colouring agent and a protective agent of the present invention.

Excrement of the cow is controlled for a few days.
a) If colour change of the excrement is observed, the diet of the animal is changed. After changing the diet of the animal, the excrement is controlled and observed normal after a few days.
b) If no colour change of the excrement is observed, the diet of the animal is not changed.

### EXAMPLE 2.

In the cow is installed a bolus, comprising a colouring agent and a protective agent of the present invention.

Excrement of the cow is controlled for a few days.
a) If colour change of the excrement is observed after installing the bolus of the invention, the diet of the animal is changed. After changing the diet of the animal, the excrement is controlled and observed normal after a few days.
b) If no colour change of the excrement is observed, the diet of the animal is not changed.

## Claims

1. A method for detecting acidosis in the rumen of a ruminant, the method comprising the following steps:
i) providing a device for a ruminant, the device comprising at least one colouring agent and a protective agent adapted to be dissolved in the rumen when the pH value of the rumen is less than 6 for at least 3 hours, so that the coloring agent is released, and
ii) detecting a visible change of the excrement when the ruminant has acidosis in the rumen.

2. A method according to claim 1, wherein the method further comprises step iii) changing the diet of the ruminant having a visible change of the excrement so as to optimize feeding of the ruminant.

3. The method according to claim 1 or 2, wherein the device is a capsule for oral administration to the ruminant, the capsule comprising a colouring agent and a protective agent which dissolves in the rumen when the pH value is less than 6 for at least 3 hours.

4. The method according to claim 1 or 2, wherein the device is a bolus for the rumen of the ruminant, the bolus comprising a colouring agent and a protective agent which dissolves in the rumen when the pH value is less than 6 for at least 3 hours.

5. The method according to claim 2, wherein changing the diet comprises at least one selected from the group consisting of changing to fiber rich diet, changing diet to bigger particle size, changing to diet containing less carbohydrates, decreasing unsaturated fats and oils, and changing the method of feeding.

6. A device for detecting acidosis in the rumen of a ruminant, wherein the device comprises
a) a colouring agent adapted to cause a visible change of the excrement, and
b) a protective agent which is adapted to be dissolved in the rumen in acidic conditions when the pH value is less than 6 for at least 3 hours, thereby releasing the colouring agent.

7. The device according to claim 6, wherein the device is a capsule for oral administration to the ruminant, the capsule comprising a colouring agent and a protective agent which dissolves in the rumen when the pH value is less than 6 for at least 3 hours.

8. The device according to claim 6, wherein the device is a bolus for the rumen of the ruminant, the bolus comprising a colouring agent, which is released to a rumen when the pH value is less than 6.

9. The method according to any one of claims 1-4 or the device according to anyone of claims 6-8, wherein the protective agent forms a coating surrounding the colouring agent(s) or the protective agent is dyed throughout with the colouring agent.

10. The method according to any one of claims 1-4 or the device according to anyone of claims 6-9, wherein the protective agent comprises a polymer, copolymer or mixture thereof.

11. The method or the device according to claim 10 wherein at least one polymer is selected from the group consisting of cellulose propionate morpholinobutyrate, and polymers, copolymers and blends of polymers selected from the group consisting of acrylonitrile, vinyl pyridine, styrene, methacrylate and methyl methacrylate.

12. Use of the device of anyone of claims 6-11 for detecting acidosis in the rumen of a ruminant.

13. Animal feed comprising the capsule of anyone of claim 7 or claims 9-11 if dependent on claim 7.

14. Animal feed according to claim 13, which is a pellet adapted for oral administration to a ruminant.

## Patentansprüche

1. Verfahren zum Feststellen von Azidose im Pansen eines Wiederkäuers, wobei das Verfahren die folgenden Schritte umfasst:
i) das Bereitstellen einer Vorrichtung für einen Wiederkäuer, wobei die Vorrichtung mindestens ein Farbmittel und ein Schutzmittel umfasst, das geeignet ist, sich im Pansen zu lösen, wenn der pH-Wert des Pansen mindestens 3 Stunden lang weniger als 6 beträgt, so dass das Farbmittel freigesetzt wird und
ii) das Feststellen einer sichtbaren Veränderung des Kots, wenn der Wiederkäuer Azidose im Pansen aufweist.

2. Verfahren nach Anspruch 1, wobei das Verfahren des Weiteren den Schritt iii) umfasst des Änderns der Ernährung des Wiederkäuers, der eine sichtbare Veränderung des Kots aufweist, so dass das Füttern des Wiederkäuers optimiert wird.

3. Verfahren nach Anspruch 1 oder 2, wobei die Vorrichtung eine Kapsel für die orale Verabreichung dem Wiederkäuer ist, wobei die Kapsel ein Farbmittel und ein Schutzmittel umfasst, das sich im Pansen löst, wenn der pH-Wert mindestens 3 Stunden lang weniger als 6 beträgt.

4. Verfahren nach Anspruch 1 oder 2, wobei die Vorrichtung ein Bolus für den Pansen des Wiederkäuers ist, wobei der Bolus ein Farbmittel und ein Schutzmittel umfasst, das sich im Pansen löst, wenn der pH-Wert mindestens 3 Stunden lang weniger als 6 beträgt.

5. Verfahren nach Anspruch 2, wobei das Ändern der Ernährung mindestens eines umfasst ausgewählt aus der Gruppe bestehend aus Übergehen zu einer ballastreichen Ernährung, Ändern der Ernährung zu einer größeren Teilchengröße, Übergehen zu einer Ernährung, die weniger Kohlehydrate enthält, Reduzieren ungesättigter Fette und Öle und Ändern des Fütterungsverfahrens.

6. Vorrichtung zum Feststellen von Azidose im Pansen eines Wiederkäuers, wobei die Vorrichtung Folgendes umfasst
a) ein Farbmittel, das geeignet ist, eine sichtbare Veränderung des Kots zu verursachen und
b) ein Schutzmittel, das geeignet ist, im Pansen unter sauren Bedingungen gelöst zu werden, wenn der pH-Wert mindestens 3 Stunden lang weniger als 6 beträgt, um dadurch das Farbmittel freizusetzen.

7. Vorrichtung nach Anspruch 6, wobei die Vorrichtung eine Kapsel für die orale Verabreichung dem Wiederkäuer ist, wobei die Kapsel ein Farbmittel und ein Schutzmittel umfasst, das sich im Pansen löst, wenn der pH-Wert mindestens 3 Stunden lang weniger als 6 beträgt.

8. Verfahren nach Anspruch 6, wobei die Vorrichtung ein Bolus für den Pansen des Wiederkäuers ist, wobei der Bolus ein Farbmittel umfasst, das in einen Pansen freigesetzt wird, wenn der pH-Wert weniger als 6 beträgt.

9. Verfahren nach einem der Ansprüche 1 - 4 oder Vorrichtung nach einem der Ansprüche 6 - 8, wobei das Schutzmittel eine Beschichtung bildet, die das/die Farbmittel umgibt, oder das Schutzmittel durchgehend mit dem Farbmittel gefärbt ist.

10. Verfahren nach einem der Ansprüche 1 - 4 oder Vorrichtung nach einem der Ansprüche 6 - 9, wobei das Schutzmittel ein Polymer, Copolymer oder eine Mischung davon umfasst.

11. Verfahren oder Vorrichtung nach Anspruch 10, wobei mindestens ein Polymer aus der Gruppe ausgewählt ist bestehend aus Cellulosepropionatmorpholinbutyrat und Polymeren, Copolymeren und Mischungen von Polymeren ausgewählt aus der Gruppe bestehend aus Acrylnitril, Vinylpyridin, Styrol, Methacrylat und Methylmethacrylat.

12. Verwendung der Vorrichtung nach einem der Ansprüche 6 - 11 zum Feststellen von Azidose im Pansen eines Wiederkäuers.

13. Tierfutter umfassend die Kapsel nach einem von Anspruch 7 oder den Ansprüchen 9 - 11, wenn sie von Anspruch 7 abhängen.

14. Tierfutter nach Anspruch 13, das ein Kügelchen ist, das für die orale Verabreichung einem Wiederkäuer geeignet ist.

## Revendications

1. Procédé de détection de l'acidose dans le rumen d'un ruminant, le procédé comprenant les étapes suivantes .
i) la fourniture d'un dispositif pour un ruminant, le dispositif comprenant au moins un agent colorant et un agent protecteur adapté pour être dissous dans le rumen lorsque le pH du rumen est inférieur à 6 pendant au moins 3 heures de sorte que l'agent colorant soit libéré, et
ii) la détection d'un changement visible de l'excrément lorsque le ruminant a une acidose dans le rumen.

2. Procédé selon la revendication 1, dans lequel le procédé comprend en outre l'étape iii) de changement du régime alimentaire du ruminant ayant un changement visible de l'excrément de façon à optimiser l'alimentation du ruminant.

3. Procédé selon la revendication 1 ou 2, dans lequel le dispositif est une capsule pour l'administration orale au ruminant, la capsule comprenant un agent colorant et un agent protecteur qui se dissout dans le rumen lorsque le pH est inférieur à 6 pendant au moins 3 heures.

4. Procédé selon la revendication 1 ou 2, dans lequel le dispositif est un bolus pour le rumen du ruminant, le bolus comprenant un agent colorant et un agent protecteur qui se dissout dans le rumen lorsque le pH est inférieur à 6 pendant au moins 3 heures.

5. Procédé selon la revendication 2, dans lequel le changement de régime alimentaire comprend au moins l'un choisi dans le groupe constitué d'un changement pour un régime riche en fibres, d'un changement de régime pour des particules de taille plus grande, d'un changement pour un régime contenant moins de glucides, de la réduction des graisses et huiles insaturées et d'un changement du procédé d'alimentation.

6. Dispositif de détection de l'acidose dans le rumen d'un ruminant, dans lequel le dispositif comprend
a) un agent colorant adapté pour entraîner un changement visible de l'excrément, et
b) un agent protecteur qui est adapté pour être dissous dans le rumen dans des conditions acides lorsque le pH est inférieur à 6 pendant au moins 3 heures, libérant ainsi l'agent colorant.

7. Dispositif selon la revendication 6, dans lequel le dispositif est une capsule pour l'administration orale au ruminant, la capsule comprenant un agent colorant et un agent protecteur qui se dissout dans le rumen lorsque le pH est inférieur à 6 pendant au moins 3 heures.

8. Dispositif selon la revendication 6, dans lequel le dispositif est un bolus pour le rumen du ruminant, le bolus comprenant un agent colorant qui est libéré dans un rumen lorsque le pH est inférieur à 6.

9. Dispositif selon l'une quelconque des revendications 1 à 4 ou dispositif selon 1"une quelconque des revendications 6 à 8, dans lequel l'agent protecteur forme un revêtement entourant le/les agent(s) colorant(s) ou l'agent protecteur est coloré complètement par l'agent colorant.

10. Procédé selon l'une quelconque des revendications 1 à 4 ou dispositif selon l'une quelconque des revendications 6 à 9, dans lequel l'agent protecteur comprend un polymère, un copolymère ou un mélange de ceux-ci.

11. Procédé ou dispositif selon la revendication 10, dans lequel au moins un polymère est choisi dans le groupe constitué du propionate de cellulose morpholinobutyrate et de polymères, de copolymères et de mélanges de polymères choisis dans le groupe constitué de l'acrylonitrile, de la vinyl-pyridine, du styrène, du méthacrylate et du méthyl-méthacrylate.

12. Utilisation du dispositif selon 1"une quelconque des revendications 6 à 11 pour détecter l'acidose dans le rumen d'un ruminant.

13. Alimentation animale comprenant la capsule selon l'une quelconque de la revendication 7 ou des revendications 9 à 11 si elle est dépendante de la revendication 7.

14. Alimentation animale selon la revendication 13, qui est un granulé adapté pour l'administration orale à un ruminant.
